Europäisches Patentamt

(19) European Patent Office

Office européen des brevets

(11) Publication number: 0 193 431
A1

# (12) EUROPEAN PATENT APPLICATION

(21) Application number: 86400197.9

(22) Date of filing: 30.01.86

(51) Int. Cl.⁴: C 12 N 5/00
C 12 P 21/00

(30) Priority: 30.01.85 US 696538

(43) Date of publication of application:
03.09.86 Bulletin 86/36

(84) Designated Contracting States:
BE CH DE FR GB IT LI NL

(71) Applicant: THE UNITED STATES OF AMERICA
represented by the Secretary U.S. Department of
Commerce
National Technical Information Service Office of
Government Inventions and Patents 5285 Port Royal
Road
Springfield, Virginia 22161(US)

(72) Inventor: Caldwell, Harlan Delano
308 Thebian Lane
Hamilton Montana 59840(US)

(72) Inventor: Hitchcock, Penny J.
340 Owings Lane
Hamilton Montana 59840(US)

(74) Representative: Mongrédien, André et al,
c/o SOCIETE DE PROTECTION DES INVENTIONS 25, rue
de Ponthieu
F-75008 Paris(FR)

(54) Monoclonal antibodies against chlamydial genus specific lipopolysaccharide.

(57) Disclosed are monoclonal antibodies which specifically
bind to the liposaccharide (LPS) epitope of the chlamydia
genus of bacteria. A method for producing these monoclonal
antibodies is also disclosed.

EP 0 193 431 A1

## Description

### Monoclonal Antibodies Against Chlamydial Genus Specific Lipopolysaccharide

The present invention is monoclonal antibody L2I-6 which specifically binds to a chlamydial genus-specific epitope. This epitope, shared by all members of the genus chlamydia, is located on the liposaccharide (LPS) antigen (the gylolipid group antigen) of these obligate intracellular procaryotes.

The chlamydial LPSs process at least three distinct antigen domains, two of which are shared by other gram-negative organisms. The third, the chlamydial genus-specific epitope recognized by the monoclonal antibody L2I-6 of the present invention, is common only to members of the genus chlamydia.

Part of the difficulty in producing monoclonal antibodies to the chlamydial LPS is that quantitative yields of chlamydial LPS are achieved only when elementary bodies (EBs) are first reduced and alkylated and then extracted by the hot phenol-water procedure. These observations are possibly peculiar to chlamydiae and may reflect the rather unique outer membrane and cell wall structure of these organisms. For example, it has been previously shown that C. trachomatis lacks detectable muramic acid, indicating the absence of a typical peptidoglycan structure. The ability of Chlamydia organisms to maintain a rigid shape in the absence of peptidoglycan is believed to occur via the major outer membrane protein. The chlamydial major outer membrane protein constitutes 65% or more of the total outer membrane protein and is known to be extensively crosslinked in the EB form of the parasite by disulfide bonds. The experiments described in the examples indicate that these covalent bonds which occur between this structural protein prevent its complete partitioning into the hot phenol phase, resulting in atypical extraction results (i.e., large interface phase). Examination of interface material revealed primarily large amounts of major outer membrane protein and LPS. Reduction of EBs with dithiothreitol significantly increases the yield of chlamydial LPS (Table 1). Chlamydial antigens are immunopathogenetic of disease in many animals and man. However, the characterization of these antigens has been, until recently, most difficult due to the inability to obtain sufficient amounts of uncontaminated antigens. The discovery of hybridema technology makes possible the preparation of monoclonal antibodies to chlamydia without the need of extensive purification of chlamydial antigens. The present invention discloses one such monoclonal antibody, L2I-6, which specifically binds to the chlamydial lipposaccharide epitope.

## Statement of Deposit

A cell line containing monoclonal antibody L2I-6 of the present invention was deposited prior to the filing date of this application in the American Type Culture Collection in Rockville, Maryland. The deposit is maintained in accordance with the Patent and Trademark prerequisites for such a deposit - maintained for a least 30 years under conditions assuring viability and available to the public upon issuance of a patent on this monoclonal antibody. The deposit number is ATCC N° HB 8705.

SR 3205 US MDT

## Description of the Figures

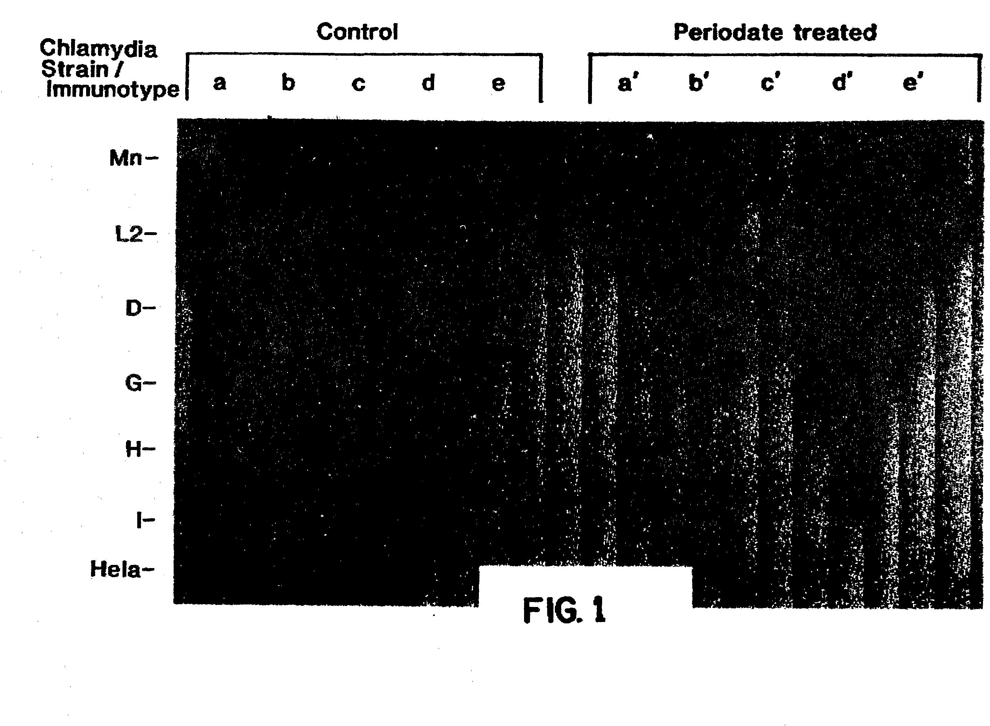

FIG. 1. Autoradiograph of monoclonal antibody-binding assay performed with hybridoma culture supernatants and chlamydia-infected HeLa 229 cells. HeLa 229 cells infected with C. psittaci Mn and C. trachomatis serovars L2, D, G, H, and I or an uninfected HeLa cell control were treated with 0.05 M sodium acetate buffer, pH 5.4 (control, lanes a through e), or with 0.05 M $NaIO_4$ in acetate buffer (periodate treated, rows a' through e'). Untreated and periodate-treated wells were reacted with monoclonal antibodies and antibody binding detected with $^{125}I$-labeled protein A. Lanes a, a', d, d', e, and e' were monoclonal antibodies that react with the major outer membrane protein of Chlamydia spp. Lanes c and c' contained hybridoma supernatant fluid that either did not react with chlamydia-infected cells or failed to bind protein A. Note that the binding of these antibodies was not affected by periodate treatment (lanes a', d', and e'). In contrast, rows b and b' contained monoclonal antibody L2I-6. Note that this antibody reacted with all chlamydial strains tested and that the antibody binding was significantly reduced by periodate treatment.

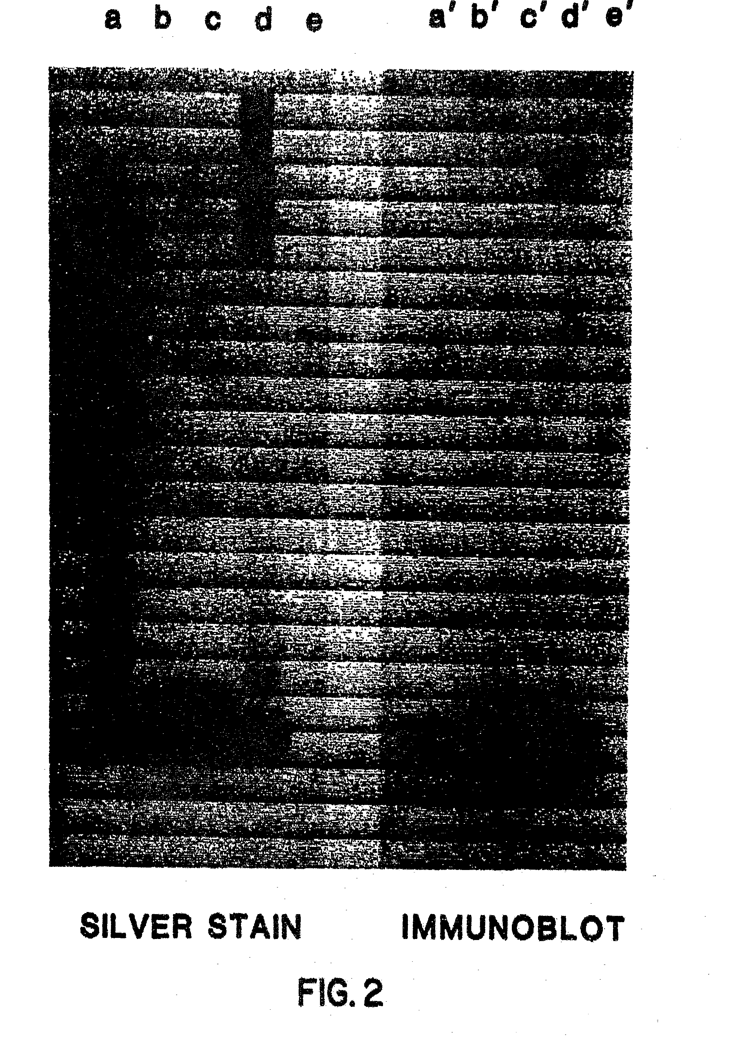

FIG. 2. Immunoblot of phenol-water-isolated chlamydial LPS reacted with monoclonal antibody L2I-6. Lanes a through e, silver-stained polyacrylamide gel; lanes a' through e', accompanying immunoblot. Lanes a and a', L2 serovar whole-cell lysate (20 µg of protein electrophoresed); lanes b and b', the same L2 serovar whole-cell lysate treated with proteinase K; lanes c and c', 5 µg of phenol-water-extracted LPS of L2

EBs; lanes d and d', L cell residue after harvesting L2 organisms; lanes e and e', uninfected L cells. Note that monoclonal antibody bounds to the chlamydial LPS isolated by the phenol-water procedure. Also note that the immunoreactivity and migration of the LPS was unaffected by proteinase K treatment; however, the non-immunoreactive polypeptides were completely digested by the protease (cf. lanes a and b). LPS was detectable in the L-cell debris recovered after harvesting chlamydiae (lanes d and d') but not in uninfected L cells (lanes e and e'). Notice that a high-molecular-weight component that reacted with monoclonal antibody L2I-6 was present in infected L cell debris (lanes d and d'). This material was not detected in whole-cell lysates of L2 EBs, purified chlamydial LPS, or uninfected L cell controls.

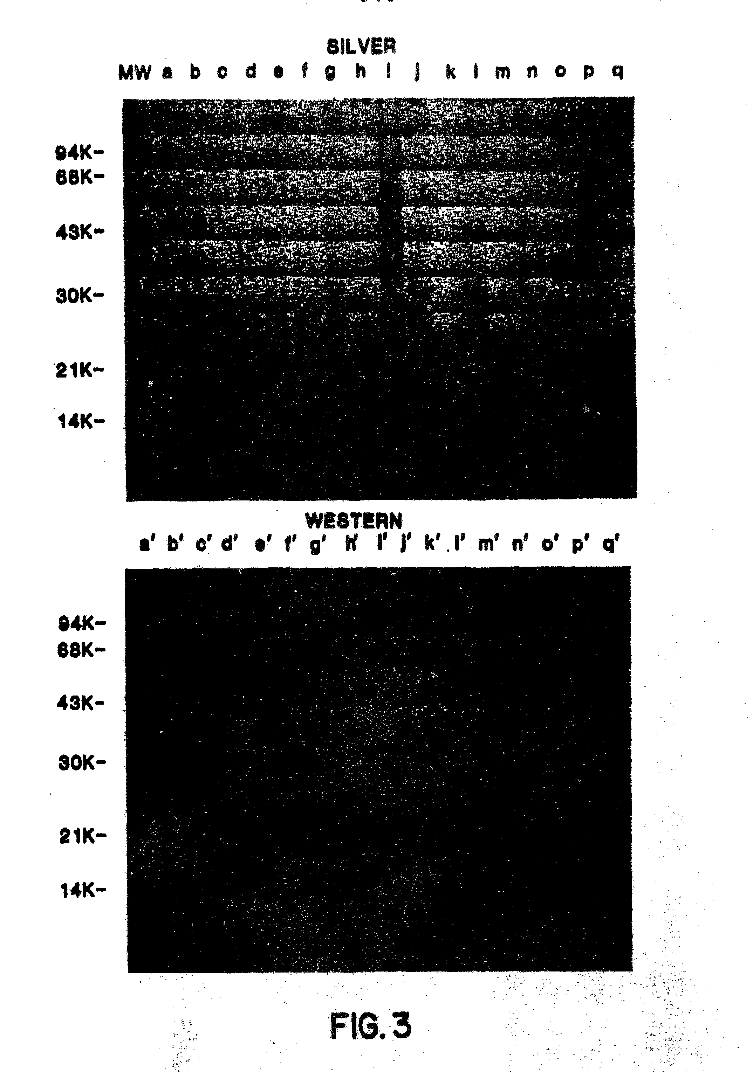

FIG. 3. Immunoblot of LPS of chlamydiae and other gram-negative organisms with monoclonal antibody L2I-6. Molecular weight (MW) standards; lanes and and a', C. trachomatis G serovar whole-cell lysate; lanes b and b', L2 serovar whole-cell lysate; lanes c and c', D serovar whole-cell lysate; lanes d and d', I serovar whole-cell lysate; lanes e and e', H serovar whole-cell lysate; lanes f and f', C. psittaci Mn whole-cell lysate; lanes g and g', N. gonorrhoeae JS1 whole-cell lysate; lanes h and h', N. gonorrhoeae JS1 LPS; lanes i and i', S. typhimurium smooth whole-cell lysate; lanes k and k', S. typhimurium chemotype $Rd_2$ LPS; lanes l and l', S. typhimurium chemotype $Rd_2$ whole-cell lysate; lanes m and m', S. typhimurium chemotype Ra LPS; lanes n and n', S. typhimurium chemotype Ra whole-cell lysate; lanes o and o', S. typhimurium chemotype Re LPS; lanes p and p', E. coli O111:B4 smooth LPS; lanes q and q', E. coli O111:B4 (J5 mutant) rough LPS. Note

SR 3205 US MDT

that the LPS of each chlamydia strain was immunoreactive with monoclonal L2I-6 (lanes a' through f'); however, none of the LPSs of the other gram-negative organisms bound monoclonal antibody L2I-6. In wells containing whole-cell lysate preparations, approximately 20  g of protein was electrophoresed. Wells with isolate LPS contained ca. 5  g (dry weight) of LPS.

## Specific Disclosure

Hybridoma supernatants produced as described in Examples 1 and 2 were screened for immunoreactivity to chlamydial antigen(s) with a solid-phase radiometric assay in which were employed HeLa 229 cells infected with the C. trachomatis serovars L2, D, and G (B serogroup) and H and I (C serogroup) and C. psittaci Mn. Hybridoma supernatants were screened in duplicate against chlamydia-infected HeLa cells that had been treated with 0.05 M $NaIO_4$ in 0.05 M acetate buffer or acetate buffer only. Treatment with $NaIO_4$ was included in the initial screening assay to identify protein A-binding monoclonal antibodies that were reactive against the periodate-sensitive antigen(s). The results of a typical assay are shown in Fig. 1. Fig. 1., lane b, the reactivity of the hybridoma L2I-6 supernatant fluid is shown. The L2I-6 monoclonal antibody reacts with HeLa cells infected with C. psittaci Mn and C. trachomatis serovars L2, D, G, H, and I, but not the infected HeLa cell control. The immunoreactivity of this group-reactive epitope is destroyed by prior treatment wiht $NaIO_4$ (Fig. 1, lane b'). The L2I-6 monoclonal antibody is an IgG3 molecule, as determined by double-immunodiffusion analyses in which subclass monospecific qoat anti-mouse

IgG antiserum is used. The genus-specific eptiope recognized by monoclonal antibody L2I-6 and a type-specific epitope located on the chlamydial major outer membrane protein appeared to be immunodominant determinants when Formalin-fixed EBs were used as the immunogen. Using the procedures described in the examples, 16 hybridomas secreting chlamydial antibodies were obtained from three different BALB/c spleen-NS1 cell fusions. Six (37%) of these hybridomas recognized the periodate-sensitive, genus-specific epitope, and 8 (48%) reacted with an L2 serovar-specific epitope located on the major outer membrane protein of the organism. All but one of these monoclonal antibodies were of the IgG3 subclass.

Location of the genus-specific epitope on chlamydial LPS. Immunoblotting analysis was used to determine whether the genus-specific epitone recognized by monoclonal antibody L2I-6 was located on chlamydial LPS (Fig. 2). The following preparations were electrophore and immunoblotted: (i) L2 EB whole-cell lysate without (lanes a and a') and with (lanes b and b') proteinase K treatment; and (ii) phenol-water-isolated chlamydial LPS (lanes c and c'), phenon-water extract of the cellular debris recovered after harvesting chlamydiae from infected L cells (lanes d and d'), and an uninfected L cell control (lanes e and e'). Several points can be made from the data presented in Fig. 2. First, the silver-stained polyacrylamide gel shows that the phenol-water isolated chlamydial LPS (lane c) is homogeneous and migrates as a low-molecular-weight molecule ($M_r$, $\leq$ 10,000). As shown in the accompanying immunoblot (lane c'), monoclonal L2I-6 intensely bounds to isolated chlamydial LPS and to material in the whole-cell and digested lysates which had similar electrophoretic mobility. These findings show that the genus-specific epitope recognized by monoclonal antibody L2I-6 is located on chlamydial LPS.

Specificity of monoclonal antibody L2I-6 for chlamydial LPS. The findings presented above show that monoclonal antibody L2I-6 recognized an epitope located on chlamydial LPS. Although preliminary data indicates that this epitope was common to the genus (Fig. 1), the reactivity of monoclonal antibody L2I-6 against each of the 15 C. trachomatis serovars and seven different C. psittaci strains were examined by microimmunofluorescence (Table 2). Antibody L2I-6 reacted at an identical end point titer of 1:320 with the acetone-fixed EBs of all chlamydiae tested. For purposes of comparison, the specificity of monoclonal antibodies L2I-45 and L2I-10 that recognize type-specific and subspecies-specific epitopes located on the major outer membrane protein, respectively, are also shown in Table 2. Monoclonal antibody H5332 was included as a negative control. These serological data show that antigenic determinant seen by antibody L2I-6 was common to chlamydial isolates of both species.

### Examples

Example 1:

Organisms. Chlamydia trachomatis was grown in HeLa 229 cells or suspension cultures of mouse L-929 cells, and EBs were purified as previously described in Caldwell et al; Infect. & Immun.; Vol. 31, pp 1161-1176 (1981).

SR 3205 US MDT

Antisera. Hyperimmune rabbit polyclonal antisera against purified EBs were prepared as previously described in Caldwell et al; Infect & Immun; Vol. 35, pp 1024-1031 (1982). The immunoglobulin G (IgG) fraction of the antisera was isolated by adsorption to protein A-Sepharose CL-4B (Sigma Chemical Co., St. Louis, Mo.).

Hybridoma production. Female BALB/c mice (3 to 4 weeks of age) were immunized with $4.0 \times 10^8$ inclusion forming units of Formalin-killed L2 EBs mixed with 0.1 ml of Freund complete adjuvant. Approximately 0.1 ml of adjuvant-EB emulsion was administered intramuscularly in a hind leg of each mouse on day 0. On days 14, 21 and 28, $4 \times 10^8$ inclusion-forming units of Formalin-killed L2 serovar was injected intravenously. On day 31, mice were sacrificed and their spleens were removed. Hybridomas of BALB/c spleen cells and murine myeloma cells (P3-NS-1-Ag-4/1) were prepared as described by Barbour et al. _J. Exp. Med._, Vol. 156, pp 1312-1324 (1982).

Example 2:

Selection and cloning hybridomas. Supernatants from wells containing growing hybridomas were assayed by a solid-phase radioimmunoassay in which chlamydial-infected HeLa 229 cells were used. Briefly, $5 \times 10^8$ 229 cells suspended in 100 1 of Eagle minimal essential medium in Hanks balanced salts and supplemented with 10% fetal bovine serum were seeded into wells of a 96-well, flat-bottomed tissue culture tray. After 24 h, the medium was removed and six rows of 12 wells were infected with Chlamydia psittaci Mn and C. trachomatis strains L2/434/Ru, D/UW-3/Cx, G/UW-57/Cx, H/UW-4/Cx, and I/UW-12/Ur. A single row of cells was not infected and served as a HeLa cell control. At 42 h postinfection, the medium was removed from wells containing cells infected with the Mn strain and the L2 serovar. These monolayers were then fixed with 1 ml of absolute methanol for 10 min and washed twice with 1 ml of 30 mM $NaPO_4$-0.15 M NaCl-0.02% $NaN_3$, DB 7.2 (phosphate-buffered saline [PBS]-azide). One milliliter of PBS-azide was added to wells containing fixed cells, and the trays were then incubated at 37° in 5% $CO_2$ for an additional 24 h. At this time, the remaining monolayers (non-LGV strains and uninfected control cells) were fixed and processed as described above.

For screening of chlamydial antibody, supernatant fluid (100 1) from wells containing proliferating hybridomas was inoculated onto fixed HeLa monolayers that were infected with the different chlamydial strains and incubated for 2 h at 37°C on an orbital shaker. The fluid was then removed, and each well was washed three times with PBS-azide for 10 min per wash. To each well 50 1 of $^{125}$I-labeled protein A (5 x $10^4$ cpm) was added, and the plates were reincubated at room temperature for 2 h on an orbital shaker. Finally, the iodinated protein A solution was removed, and the wells were washed as described above. The plate was then subjected to autoradiography with Kodak X-Omat AR film and a Lightning-Plus intensifying screen for 12 to 18 h at -68°C. Wells demonstrating bound protein A were identified, and the respective supernatant fluids were reassayed by immunoblotting to identify the immunoreactive component (see below). Hybridomas of interest were then cloned twice by limiting dilution. Monoclonal antibodies were typed by double immunodiffusion with subclass-specific goat anti-mouse InG antisera.

Example 3:

The effect of periodate treatment on chlamydial antigens was determined by the solid-phase infected-cell assay described above. Methanol-fixed, infected HeLa 229 cell monolayers were treated with 100 1 of 0.05 M sodium metaperiodate in 0.05 M sodium acetate buffer (pH 5.5) or buffer alone a 4°C for 24 h. The wells were washed with PBS-azide and probed with antibody and $^{125}I$-labeled protein A as described above. Susceptibility to periodate oxidation was considered positive when periodate pretreatment inhibited or greatly reduced monoclonal antibody binding to chlamydia-infected cells as determined by exposure of the autoradiograms.

The microimmunofluorescence method of Wang, Excerpts, Medica, R.L. Nichols (ed) pp 273-288 (1971), developed for the serilogical classification of C. trachomatis, was used to determine the chlamydial specificity of monoclonal antibodies.

Isolation of chlamydial LPS. EBs were harvested from L-929 cells 42 h after infection. Purified EBs were washed twice with PBS, and the pellet was resuspended in glass distilled water and lyophilized. The dry weight of L2 EBs obtained from a typical harvest of $2 \times 10^9$ infected L-929 cells was between 60 and 65 mg. EBs (60 mg [dry weight]) were initially extracted by the PCP method of Galanos et al, Eur. J. Biochem, Vol. 9, pp 245-249 (1969), followed by re-extracting the PCP residue by the hot phenol-water method of Westphal and Jann, Methods Carbohydr Chem., Vol. 5, pp 83-91 (1965). The procedure was modified in that ultracentrifugation was extended from 3 to 16 h. Subsequently, EBs were reduced with 50 mM dithiothreitol (Sigma) in 20 ml of PBS for 30 min at 4°C. One milliliter of 1.0 M iodoacetamide in PBS was added, and the mixture was kept at 4°C for 1 h. The reduced and alkylated EB suspension was centrifuged at 30,000 x g for 15 min at 4°C, and the EB pellet was resuspended in 10 ml of cold distilled water. This suspension was then lyophilized and weighed. The LPS from 32.6 mg (dry weight) of EBs treated in this manner was readily extracted by the hot phenol-water method. The length of ultracentrifugation was again increased to 16 h.

Example 4:

Immunoblotting. An immunoblot procedure was used in which electrophoretic transfer was done in 25 mM sodium phoshate (pH 7.3) at 27 V (0.9 to 1.0 A) for 2 h at 17°C to HAHY Millipore nitrocellulose paper with a Trans-Blot cell (Bio-Rad Laboratories, Richmond, Calif.). After transfer, the nitrocellulose paper was incubated in 50 mM sodium phosphate-0.15 M NaClO-0.02% NaN$_3$ containing 3% bovine serum albumin for 30 min. The nitrocellulose paper was then incubated with antiserum or monoclonal antibody diluted in PBS-bovine serum albumin for 2 to 16 h on a rocker platform (Rellco). The nitrocellulose paper was washed three times for 5 to 15 min with PBS, followed by incubation with $^{125}$I-labeled protein A (5 x 10$^5$ cpm/ml) for 1 h at room temperature. The nitrocellulose paper was then washed in PBS, pressed between sponges, and air dried at 37°C. Chlamydial LPS bound very poorly to

SR 3205 US MDT

nitrocellulose paper when buffers containing detergents (Tween-20 or Nonidet P-40) were used, and therefore, detergent was omitted from all immunoblotting procedures. Autoradiography was done with Kodak x-Omat AR film with a Lightning-Plus intensifying screen at -68°C for 4 to 12 h.

Example 5:

Isolation of chlamydial LPSs. The $NaIO_4$ sensitivity and the genus prevalence of the epitope recognized by monoclonal antibody L2I-6 indicates that the antigen is the chlamydial glycolipid antigen. Since the chlamydial glycolipid antigen is an LPS molecule, this example describes the isolation of the glycolipid antigen with LPS extraction procedures. Purified EBs were first treated with dithiothreitol, blocked reduced sulfhydryls with iodoacetamide, and then subjected these preparations to both LPS extraction procedures. A total of 0.625 mg of LPS (1.9% yield) was recovered from 32.6 mg of reduced and alkylated EBs by the phenol-water procedure (Table 1). However, even using reduced and alkylated EBs, we were unable to extract LPS by the Galanos procedure. Characterization of chlamydial LPS is summarized in Table 1. Parallel analyses of S. typhimurium Re LPS were done. Both LPSs and similar activities in a Limulus amoebocyte lysate assay. The KDO values were dissimilar. Although reproducible analytical figures were obtained, they did not indicate the true KDO content of the samples. Not only is KDO labile, it is known that the molar absorption of KDO varies considerably with the presence of substituent (11). The low values obtained for total carbohydrates in chlamydial and Re LPSs by the phenol-sulfuric acid method were at or below the limits of reliability for the assay. The test results were indicative of low hexose contents in both LPSs.

Example 6:

In order to determine whether this epitope was chlamydia specific or a determinant present on the LPS of other gram-negative organisms, the immunoreactivity of antibody L2I-6 against six chlamydial isolates, N. gonorrhoeae JS1, S. typhimurium, and Escherichia coli was tested by immunoblotting. Both whole-cell lysate preparations and isolated LPS were analyzed for each organism. Additionally, several S. typhimurium strains which represented different LPS chemotypes were tested.

These were S. typhimurium smooth, Ra, $Rd_2$, and Re LPSs. Isolated smooth and rough LPS of E. coli 0111:B4 and mutant J5 were also tested. The silver-stained profiles and their accompany immunoblot against monoclonal antibody L2I-6 are shown in Fig. 3. The LPS of each chlamydial strain reacted strongly with this antibody (lanes a' through f'); however, LPSs from N. gonorrhoeae, S. typhimurium, and E. coli were not reactive (lanes g' through q'). In addition, isolated LPSs of Coxiella burnetii (phase I or II) and Rickettsia rickettsii did not react with antibody L2I-6 by immunoblotting. These immunological findings show that, at least for the limited number of extracellular and obligate intracellular bacteria examined, the genus-specific epitope recognized by monoclonal antibody L2I-6 is unique to chlamydiae and is not shared by the LPS of other gram-negative bacteria.

Table 1

Characterization and comparison of Chlamydia and Salmonella RE LPS

| LPS | Starting Material | Extraction method[a] (%)[e] | Yield (%) | Protein (%)[b] | KDO (%)[c] | LAL test result[d] | Total hexoses and pentoses | Reactivity with | | |
|---|---|---|---|---|---|---|---|---|---|---|
| | | | | | | | | L2I-6[f] | Anti-Re serum | Anti-lipid A serum |
| C. trachomatis L2 | Reduced and alkylated EBs | P-W | 1.9 | <1.0 | 8.8 | + | 10.5 | + | + | + |
| S. typhimurium LT[2] Re mutant | SAI no. 377 Re whole cells | PCP | 3.0 | 2-3.0 | 19.3 | + | 5.0 | - | + | + |

[a] P-W, Phenol-water (see text).

[b] Percent protein was determined on a weight basis by the method of Lowry et al. (21).

[c] Percent KDO was determined on a weight basis (11,25).

[d] LAL, Limulus amoebocyte lysate (Difco). Both LPSs were positive at >0.1 ng/ml.

[e] Numbers represent the means of values obtained in two separate experiments and determined by the phenol-sulfuric acid method of Dubois et al. (14). This procedure is suitable for the estimation of total carbohydrates in bacterial cells that contain >10% hexose polymers. The pentoses in nucleic acid interfere when hexose content is low. The difference between total hexoses and pentoses of S. typhimurium Re mutant and C. trachomatis cannot, therefore, be regarded as significant.

[f] Monoclonal antibody L2I-6 reacted with the genus-specific LPS epitope.

Table 2

## Specificity of monoclonal antibody L2I-6 by microimmunofluorescence

| Chlamydia serovar or strain | Monoclonal antibody micro-IF titer[a] | | | |
|---|---|---|---|---|
| | L2I-6 | L2I-45 | L2I-10 | H5332 |
| C. trachomatis serovar | | | | |
| A | 320 | – | – | – |
| B | 320 | – | – | – |
| Ba | 320 | – | 160 | – |
| C | 320 | – | – | – |
| D | 320 | – | 160 | – |
| E | 320 | – | 160 | – |
| F | 320 | – | 80 | – |
| G | 320 | – | 80 | – |
| H | 320 | – | – | – |
| I | 320 | – | – | – |
| J | 320 | – | – | – |
| K | 320 | – | 80 | – |
| L1 | 320 | – | 160 | – |
| L2 | 320 | 320 | 160 | – |
| L3 | 320 | – | 160 | – |
| Mouse pneumonitis | 320 | – | – | – |
| | | | | |
| C. psittaci strain | | | | |
| Bovine abortion 8390 | 320 | – | – | – |
| Feline pneumonitis 562 | 320 | – | – | – |
| Guinea pig inclusion conjunctivitis | 320 | – | – | – |
| Meningopneumonitis Cal-10 | 320 | – | – | – |
| 6BC | 320 | – | – | – |
| Parrot/Meyer | 320 | – | – | – |
| Parakeet/Arizona | 320 | – | – | – |

[a] Reciprocal of highest dilution resulting in positive microimmunofluorescence (micro-IF). L2I-6, Chlamydial LPS; L2I-45, major outer membrane protein L2 serovar-specific epitope; L2I-10, major outer membrane protein C. trachomatis subspecies epitope; H5332, negative control monoclonal antibody against an outer membrane protein of the Lyme disease spirochete. –, Negative at a dilution of 1:10.

## Claims:

1. Cell line containing monoclonal antibody L2I-6, and characterized essentially by containing monoclonal antibodies which specifically bind to the lipopolysaccharide epitope on chlamydial trachomatis.

2. The cell line of Claim 1 wherein said monoclonal antibodies are genus-specific for the chlamydial lipopolysaccharide epitope.

FIG. 1

Chlamydia Strain / Immunotype

Control: a  b  c  d  e

Periodate treated: a'  b'  c'  d'  e'

Mn–
L2–
D–
G–
H–
I–
Hela–

1/3

0193431

a    b    c    d    e        a'  b'  c'  d'  e'

SILVER STAIN        IMMUNOBLOT

FIG. 2

SILVER

MW a b c d e f g h i j k l m n o p q

94K-
68K-

43K-

30K-

21K-

14K-

WESTERN

a' b' c' d' e' f' g' h' i' j' k' l' m' n' o' p' q'

94K-
68K-

43K-

30K-

21K-

14K-

FIG. 3

# 0193431

## EUROPEAN SEARCH REPORT

Application number

EP 86 40 0197

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 101, no. 1, 2nd July 1984, page 451, no. 5208n, Columbus, Ohio, US; H.D. CALDWELL et al.: "Monoclonal antibody against a genus-specific antigen of Chlamydia species: location of the epitope on chlamydial lipopolysaccharide", & INFECT. IMMUN. 1984, 44(2), 306-14 * Abstract * | 1,2 | C 12 P 21/00 C 12 N 5/00 |
| X | CHEMICAL ABSTRACTS, vol. 102, no. 25, 24th June 1985, page 466, no. 219404t, Columbus, Ohio, US; M.J. THORNLEY et al.: "Properties of monoclonal antibodies to the genus-specific antigen of Chlamydia and their use for antigen detection by reverse passive hemagglutination", & J. GEN. MICROBIOL. 1985, 131(1), 7-15 * Abstract * | 1,2 | |
| | --- -/- | | |

**TECHNICAL FIELDS SEARCHED (Int. Cl.4)**

A 61 K
C 12 P
C 12 N

The present search report has been drawn up for all claims

| Place of search THE HAGUE | Date of completion of the search 03-06-1986 | Examiner CHARLES D.J.P.I.G. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 102, no. 1, 7th January 1985, page 409, no. 4282r, Columbus, Ohio, US; R. PEELING et al.: "In vitro neutralization of Chlamydia trachomatis with monoclonal antibody to an epitope on the major outer membrane protein", & INFECT. IMMUN. 1984, 46(2), 484-8 * Abstract * | 1,2 | |
| A | JOURNAL OF IMMUNOLOGY, vol. 128, no. 3, March 1982, pages 1083-1089, The American Association of Immunologists, Baltimore, Maryland, US; R.S. STEPHENS et al.: "Monoclonal antibodies to Chlamydia trachomatis: Antibody specificities and antigen characterization" * Page 1083, right-hand column, lines 15-34, 49-52; page 1085, left-hand column, line 6 - page 1087, left-hand column, line 14; page 1088, right-hand column, lines 21-38 * | 1,2 | TECHNICAL FIELDS SEARCHED (Int. Cl.4) |

--- -/-

The present search report has been drawn up for all claims

| Place of search | Date of completion of the search | Examiner |
|---|---|---|
| THE HAGUE | 03-06-1986 | CHARLES D.J.P.I.G. |

| | DOCUMENTS CONSIDERED TO BE RELEVANT | | Page 3 |

| Category | Citation of document with indication, where appropriate, of relevant passages | Relevant to claim | CLASSIFICATION OF THE APPLICATION (Int. Cl.4) |
|---|---|---|---|
| A | JOURNAL OF IMMUNOLOGY, vol. 130, no. 6, June 1983, pages 2899-2902, The American Association of Immunologists, Baltimore, Maryland, US; A. ROSEN et al.: "Human monoclonal antibodies to a genus-specific Chlamydial antigen, produced by EBV-transformed B cells" * Page 2900, right-hand column, lines 17-21, 50-65; page 2902, left-hand column, table I * | 1,2 | |
| A | WO-A-8 303 678 (HYBRITECH INC.) * Page 1, lines 2-4; page 10, example 3 and line 20 - page 12, line 18 * | 1,2 | TECHNICAL FIELDS SEARCHED (Int. Cl.4) |

| The present search report has been drawn up for all claims | | |
|---|---|---|
| Place of search THE HAGUE | Date of completion of the search 03-06-1986 | Examiner CHARLES D.J.P.I.G. |